# EUROPEAN PATENT APPLICATION

(11) **EP 2 020 191 A2**
(43) Date of publication of application: **04.02.2009**
(21) Application number: 08252582.5
(22) Date of filing: 30.07.2008
(51) Int. Cl.: A45D 34/04, A61B 17/54

(54) **Mechanical skin resurfacing device and method for use**

(30) Priority: 31.07.2007 US 953068 P
(71) Applicant: Johnson and Johnson Consumer Companies, Inc., Skillman, NJ 08558 (US)
(72) Inventor: Gubernick, David, Cherry Hill, NJ 08003 (US); Hull, Raymond J., Jr., Hampton, NJ 08827 (US); Menke, James, Califon, NJ 07830 (US); Rytel, John F., East Brunswick, NJ 08816 (US)
(74) Representative: Kirsch, Susan Edith

(57) **Abstract**

A method for resurfacing the skin includes imparting sufficient energy to a motorized device (3) to cause a mass (23) of less than 4 grams to move in an eccentric orbit, thereby urging an abrasive (11) coupled to said device to vibrate; and contacting said skin with said vibrating abrasive.

## Description

### BACKGROUND OF THE INVENTION

Numerous techniques have been proposed to provide cosmetic and/or or skin rejuvenation benefits. One of the more popular techniques, professional microdermabrasion, is a non-invasive procedure in which a device pulls the skin via suction and bombards the skin with abrasive particles in order to affect an exfoliation. Professional microdermabrasion devices, however, are cumbersome in that they occupy a large amount of space and also require a high power input and must be plugged into an AC outlet during operation. Furthermore, the patient must make regular visits to the professional skin care specialist where he or she receives treatment. Accordingly, "at home" microdermabrasion systems that combine a motorized apparatus and an abrasive system are now available.

Rhoades, US Pat. No. 6652888, purports to disclose a method for skin rejuvenation with buffing cream In this method, a cream moisturizer for resurfacing human skin is provided with a suspension of microcrystals of alumina in a ratio of approximately 14 grams per ounce of moisturizer cream. The cream moisturizer is buffed into the epidermal layer of the skin with a closed-cell sponge pad driven by a vibrator. The alumina microcrystals buffs an epidermal layer off the skin to provide a soft smooth surface, thereby rejuvenating the skin.

Dotan, US Pat. No. 6,139,553, purports to disclose a facial treatment implement and method. In this disclosure, an implement for and a method of, facial treatment are described in which a facial preparation containing an abrasive is applied to the face, and a facial treatment head is pressed against the facial preparation and is vibrated to work the facial preparation into the skin of the subject's face. Preferably, the facial treatment head includes a convexly-curved surface, but may also be one including a rotary disk which is rotated and vibrated while pressed into contact with the facial preparation on the subject's face. The facial preparation is preferably a mud mixture; preferably, this treatment is followed by another one utilizing a facial preparation containing a moisturizer which is also worked into the skin in a similar manner as the mud treatment.

Zelickson, US Pat. No. 6,645,184 purports to disclose a tape stripping system and method. The system uses a tape-like material by applying it to human skin and then stripping the tape from the skin to remove excess or unwanted material. The system further includes application of other elements to facilitate skin recovery and rejuvenation processes.

We have recognized that while "at-home" microdermabrasion systems are commercially available, these systems, while efficacious, may be less than optimal for various reasons. Concerns that smaller devices are less effective has kept these devices rather large. However, we have also recognized that available "at-home" microdermabrasion systems are often somewhat heavy and/or bulky and therefore difficult to manuever, obscure one's ability to see the skin surface being treated and have unnecessarily high packaging costs. We have further recognized the need to overcome one or more of the above mentioned drawbacks, yet still be able to maintain the efficacy of skin treatment.

### SUMMARY OF THE INVENTION

In one aspect, a method for resurfacing the skin is provided. The method includes imparting sufficient energy to a micromotorized device to cause a mass of less than 4 grams to move in an eccentric orbit, thereby urging an abrasive coupled to said device to vibrate. The method further includes placing the vibrating abrasive in contact with said skin.

### BRIEF DESCRIPTION OF THE DRAWINGS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.
Figure 1 is a schematic side view of a system for treating the skin that is consistent with embodiments of the invention described herein;
Figure 2 is a front view of the system of Figure 1;
Figure 3 is a top view of a skin-contactable element suitable for use with the system of Figure 1;
Figure 4 is a cross sectional view of Figure 1, taken through line 1-1'; and
Figure 5 is a perspective view of a motor suitable for use in the system of Figure 1.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

It is believed that one skilled in the art can, based upon the description herein, utilize the present invention to its fullest extent. The following specific embodiments are to be construed as merely illustrative and not limiting the remainder of the disclosure in any way whatsoever.

As used herein the specification and the claims, the term "mechanical skin resurfacing technique" and variants thereof relate to the mechanically assisted removal of mammalian (especially human) skin cells, ranging from mild techniques (such as exfoliation and abrasive cleansing) through microdermabrasion, and up to severe techniques such as dermal abrasion.

As used herein the specification and the claims, the term "dermabrasion" and variants thereof relate to a non-thermal resurfacing technique especially well suited for deep defects of the skin such as acne scars, heavy wrinkles and the disfiguring effects of skin conditions like rosacea. The procedure involves the mechanical sanding of the upper layers of the skin and penetrates the skin deeper than microdermabrasion. With dermabrasion, a new layer of skin replaces the abraded skin during healing, resulting in a smoother appearance

As used herein the specification and the claims, the term "microdermabrasion" and variants thereof relate to a very mild and less-penetrating form of dermabrasion, more suited for reduction of fine lines and wrinkles and for other less severe skin conditions. Microdermabrasion penetrates less deeply into the skin, primarily the stratum corneum, or portions thereof.

As used herein the specification and the claims, the term "exfoliation" and variants thereof relate to the peeling and sloughing off of the skin's tissue cells.

As used herein the specification and the claims, the term "cleansing" and variants thereof relate to removal of dirt, oils, and the like from the surface of the skin, especially through surfactant washing, and perhaps also penetrating into the pores of the skin. In "abrasive cleansing," some degree of exfoliation also occurs.

These mechanical skin treatments may facilitate the delivery of benefit agents to skin tissue, e.g., cleansing and the delivery of acne treatment compositions or rejuvenating agents such as retinol.

As used herein the specification and the claims, the term "nonwoven" and variants thereof relate to a sheet, web, or batt of natural and/or man-made fibers or filaments, excluding paper, that have not been converted into yarns, and that are bonded to each other by any of several means. For additional clarification, nonwovens are distinct from woven and knitted fabrics. The fibers included in the nonwoven materials may be staple or continuous or be formed in situ, and preferably, at least about 50% of the fibrous mass is provided by fibers having a length to diameter ratio greater than about 300:1.

The present invention is directed to systems, articles, compositions, and methods useful for mechanical skin resurfacing techniques employing a handheld motorized device. In various embodiments of the invention, such systems, articles, and methods provide a unique combination of high reliability and convenience for the user, as well as a highly efficacious mechanical skin resurfacing technique.

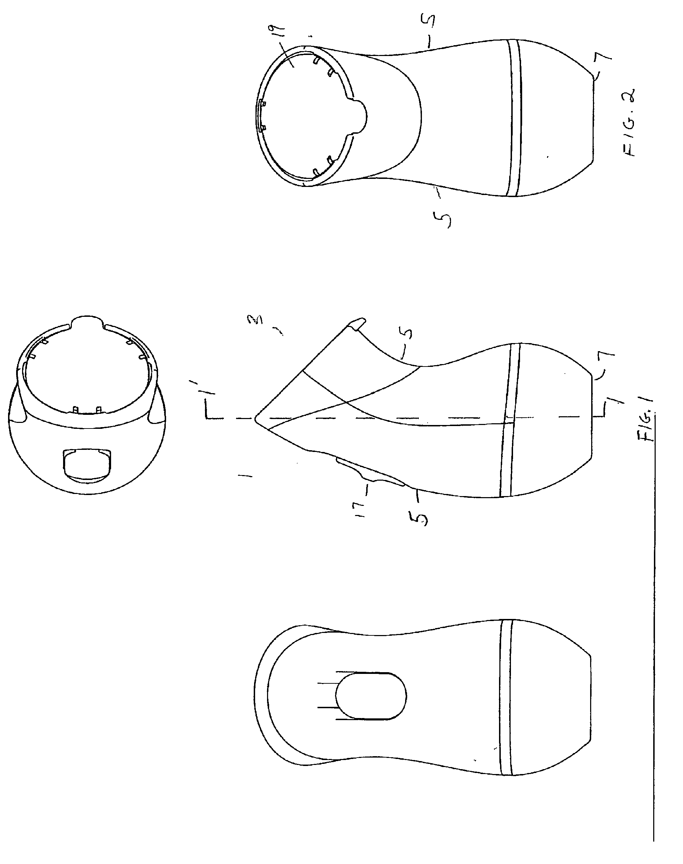

Figure 1 depicts a side view of one non-limiting example of a system 1 useful for mechanical skin resurfacing according to embodiments of invention described herein. The system 1 includes a motorized apparatus 3 that is generally shaped to be held in a hand of a user.

The apparatus 3 may be of varying shapes and dimensions, and one notable shape is a curved tubular shape that includes a convex gripping surface 5. The convex gripping surface 5 bulges out away from the volumetric center of the apparatus 3, terminating in a basal surface 7.

Figure 2 depicts a front view of system 1. The apparatus 3 may include one or more surfaces 19 for removably attaching a skin-contactable element thereto. The term, "removably attaching," and variants thereof, relate to the ability to attach, remove, and reattach the element without significantly compromising the attachment strength.

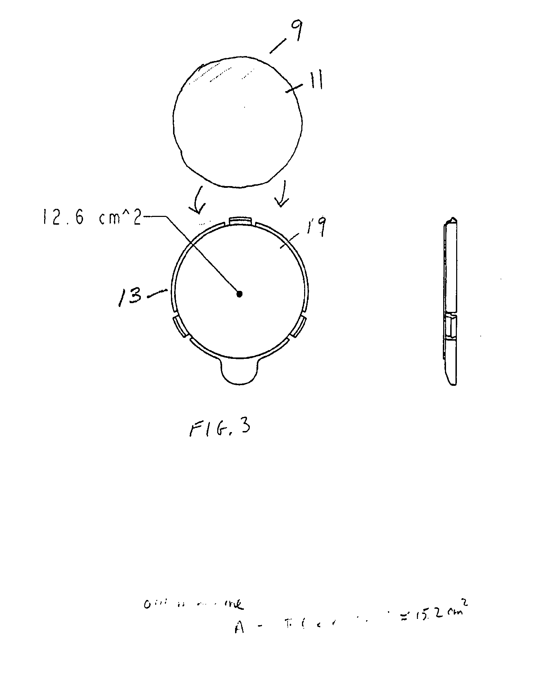

Figure 3 depicts a top view of a skin-contactable element 9 (e.g., a free-standing mass such as a sponge, a fibrous material or other material, or combinations thereof, including those described in this specification, below) that includes a skin-contactable surface 11 for contacting the skin. The skin-contactable element 9 may be placed (as indicated by the arrows in Figure 3) onto and secured to an optional carrier 13 (e.g., a firm plastic substrate). The skin-contactable element 9 may be secured to the carrier by any of various means, e.g., microhooks, adhesive, and the like. The carrier 13 may be removably attached and detached from the one or more surfaces 19 of the apparatus 3 via snap, threaded screw, friction fit or otherwise.

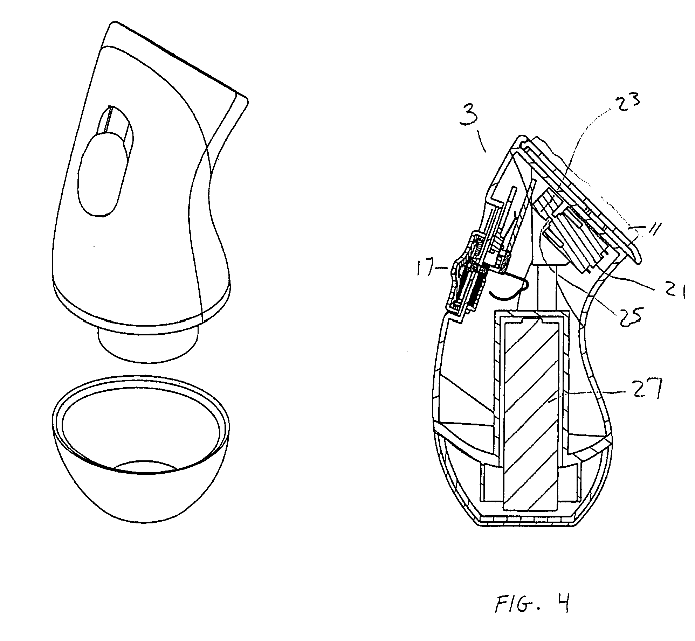

Figure 4 depicts a cross-sectional view taken through line 1-1' shown in phantom in Figure 1. A user grasping the gripping surface 5 activates a motor 21 within the apparatus 3, such as by actuating a switch 17 on the apparatus 3, allowing stored energy from a battery 27 to energize the motor 21. The motor 21, thereby enregized, provides mechanical energy that is transmitted to the attached skin-contactable surface 11 and to an expanse of skin (not shown) placed in contact therewith. The mechanical energy comprises a vibrational form that is transmitted via an eccentric weight 23 on a rotating shaft 25. The gripping surface 5 is generally shaped to facilitate easy grasping by the user so that the apparatus 3 is oriented such that the attached vibrating skin-contactable surface 11 can contact the user's skin.

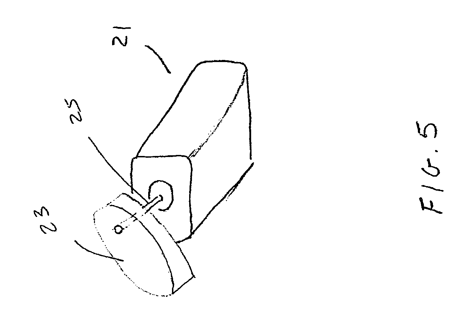

In order facilitate the transmission of energy from the motor to the skin-contactable element 9, the motor 21 may be positioned in close proximity to the skin-contactable element 9 (Figure 5 shows a perspective view of a suitable motor). While the axis of the shaft 25 is desirably parallel to the skin-contactable surface 11, as shown in Figure 4, this is not required.

Applicants have surprisingly found that the vibrational energy provided by the apparatus 3 need not be high in order to provide skin benefits. In particular, Applicants have found that a mass of less than 4 grams provides is capable of providing sufficient microdermabrasion when used with an abrasive. In one embodiment, the mass of the eccentric weight is less than about 3 grams. In another embodiment, the mass of the eccentric is less than about 2 grams. In another embodiment, the mass of the eccentric weight is from about 1.6 grams to about 4 grams.

The inventors have found that by reducing the mass of the eccentric weight 23, not only can one achieve microdermabrasion, but one can adjust the design of the apparatus to achieve other benefits as well. Specifically by reducing the mass of the eccentric weight 23, (1) one can reduce the space occupied by the motor and reduce the space occupied by the entire apparatus, making it easier to see areas of the skin when the apparatus is in use; (2) the apparatus can be made easier to transport; (3) the apparatus can be made such that is has reduced packaging costs; and (4) one can also reduce the weight of the apparatus 3 making it lighter and less tedious to hold for extended use periods.

According to embodiment of the invention, vibrating abrasive is placed in contact with the skin to be treated. While the above Figures depict the abrasive as an abrasive skin-contactable element, this is not required. The abrasive may be loose abrasive particles, e.g, mineral particles such as those including oxides of aluminum, mica, silicates and the like, as well as organic abrasive particles such as ground walnut shells, ground apricot shells, ground inorganic particles.

In order to avoid difficulties in removing/rinsing loose abrasive particles after use, in a preferred embodiment, the abrasive is an abrasive, skin-contactable element, such as the skin-contactable element 9 described in relation to the above-mentioned Figures. The abrasive, skin-contactable element skin free-standing mass such as a sponge, a fibrous material or other material, or combinations thereof that has inherent abrasiveness or has a abrasive system bound thereto.

The abrasive skin-contactable element 9 may comprise, consists essentially of, or consists of a fibrous material. Suitable fibrous materials include, without limitation, woven, nonwoven (oriented, e.g., via a carding process, or non-oriented), or knit fabrics. The fibers may be integrated into a nonwoven structure via, for example, needle punching, through-air bonding, hydro entangling, spun-bonding, chemical bonding (including adhesive bonding), or mechanical processing (such as embossing).

The fibers may thereby be arranged into a freestanding fabric (e.g., a porous fabric). The nonwoven fabric may have an average pore diameter (as calculated via Cohen, "A Wet Pore-Size Model for Coverstock Fabrics," Book of Papers: The International Nonwovens Fabrics Industry, pp.317-330, 1990) that is from about 150 microns to about 500 microns, such as from about 220 microns to about 400 microns. A representative, non-limiting list of useful fibers includes fibers derived from organic polymers such as, for example, polyester, polyolefin, polyamide and rayon fibers and bicomponent fibers; cellulose-based fibers such as wood pulp, rayon, and cotton; and combinations thereof

In order to provide a particularly suitable degree of abrasion, the skin-contactable element 9 may include fibers are bonded via mechanical means such as a needle-punching process, known to those skilled in the art, such as to a thickness of about 0.5mm to about 5mm, more preferably from about 1mm to about 5mm. The fibrous material may have a basis weight (mass per unit area) sufficient to maintain its mechanical integrity for one or more uses of the skin-contactable element 29. The basis weight may be, for example, between about 10 grams per square meter (gsm) and about 450 gsm, such as between about 200 gsm and about 400 gsm, preferably between about 300 and about 400 gsm. The fibrous material desirably includes rayon to provide softness and a strong, resilient material such as an olefin or polyester. One particularly notable fibrous material is a needle-punched blend of staple-length 1.5 denier "TENCEL" rayon and staple-length 4-5 denier PET available from Precision Custom Coating of Totowa, NJ, with a basis weight of about 200 gsm and about 400 gsm.

The bound abrasive may be an abrasive system that is bound to fibers. The term an abrasive system "bound to fibers" refers to abrasive units, particles, aggregates, and the like that are firmly attached to the fibers and do not readily separate in use therefrom. Such abrasive may be bound by various means; one notable means is by chemical bonding (including, without limitation, adhesive bonding).

The abrasive system may include or consist essentially of a water insoluble abrasive material. In one embodiment of the invention, the abrasive system includes a resin or polymer. For example, the polymer may be a homopolymer, copolymer, or terpolymer, and may be a blend of two or more different polymers. The polymers may be random, block, star, or other known architecture. The polymer may be made by known means, such as emulsion polymerization, dispersion, suspension, or solution polymerization. In a preferred embodiment the polymer is formed by emulsion polymerization. The polymers may be non-functional, or may contain functionality designed to optimize the properties of the coating in the specific application. One of skill in the art will be able to adjust monomer content and architecture to improve end-use performance of the polymer composition. The polymer could be a synthetic polymer, or could be a natural polymer such as, for example, a polysaccharide, starch, modified starch, or guar gum. Preferred polymers include homopolymers and copolymers having one or more of the following monomers: (meth)acrylates, maleates, (meth)acrylamides, vinyl esters, itaconates, styrenics, unsaturated hydrocarbons and acrylonitrile, nitrogen functional monomers, vinyl esters, alcohol functional monomers. Particularly preferred monomers include, but are not limited to, vinyl acetate; methyl (meth)acrylate, ethyl (meth)acrylate, butyl (meth)acrylate, ethylene, vinyl chloride, and styrene.

If included in the skin-contactable element,, the polymer is selected so as to provide enough hardness so as to be abrasive to skin, but not so hard as to cause scratching or discomfort. In one embodiment of the invention, the polymer has a glass transition temperature, T_{g} greater than about -20 degrees Celsius (° C.), such as from about 0° C. to about 105° C. In one notable embodiment, the polymer has a T_{g} from about 0° C and about 50° C.

T_{g} can be determined by differential scanning calorimetry (DSC) conducted at a heating rate of 20.0 ° C./minute with 5 mg or smaller samples. The T_{g} is calculated as the midpoint between the onset and endset of heat flow change corresponding to the glass transition on the DSC heat capacity heating curve. The use of DSC to determine T_{g} is well known in the art, and is described by B. Cassel and M. P. DiVito in "Use of DSC To Obtain Accurate Thermodynamic and Kinetic Data", American Laboratory, January 1994, pp 14-19, and by B. Wunderlich in Thermal Analysis. Academic Press, Inc., 1990.

The polymer may be a thermosetting polymer, (e.g., a polymer having crosslinks that are generally not reversible with changes in temperature). One notable polymer has an acrylic base/ vinyl acrylic base that is partially cross-linked during cure with a Tg of about 30° C., e.g., VINAMUL ABX 30, resin commercially available as from Celanese Corporation of Dallas, TX.

The abrasive system may further include one or more additional functional components compounded with the abrasive. Useful additional functional components include, but are not limited to plasticizers; cross-linkers; starch; polyvinyl alcohol; formaldehyde thermosetting agents such as melamine, urea, phenol; fillers; humectants; surfactants; salts; fragrances; and pigments or reflective agents. The additional functional components may be present in the abrasive system at from 0 to 20 percent by weight, and preferably from 5 to 15 percent by weight, calculated as a percent of the polymer solids.

The skin-contactable element 49 may be formed by depositing the abrasive system 43 onto the fibers 45 by various means known to the art of industrial polymer coating, such as slot coating, foam coating, saturation, printing, or spraying. Spraying is particularly notable to facilitate the formation of discrete abrasive units on top of the fibers so that waste is reduced and efficacy is optimized. If the abrasive system is applied by spraying, a sprayable composition that includes the abrasive system (e.g., polymer plus other functional ingredients as well as water or another suitable carrier) may be sprayed onto the fibers followed by drying the resulting fiber/abrasive composite in a conventional oven.

Although, the foregoing relates to a skin-contactable element that incorporates an abrasive system bound to the fibers, in one embodiment of the invention, the fibers themselves may be abrasive, without the need of including an additional abrasive system bound to the fibers. For example, in one particular embodiment, the skin-contactable element includes staple fibers that are integrated into a nonwoven structure via needle punching, through air bonding, or thermal bonding. The fibers may be high denier fibers formed from polyester; polyolefins; rayon fibers; bicomponent fibers; cellulose-based fibers such as wood pulp, rayon, and cotton; or combinations thereof.

The inventors have surprisingly found that one or more of the above-mentioned desirable attributes may be achieved by using moderately abrasive skin-contactable elements that have an appropriate "Durable Abrasiveness" determined according to the "Durable Abrasiveness Test" described in the "Test Methods" section, below. Furthermore, the above-mentioned benefits are further enhanced if the skin-contactable elements are selected based upon their Durable Abrasiveness in combination with one or more properties relating to how the pads behave under compressive load, specifically Compressibility and Displacement. These properties relate to the ability of the skin-contactable element to transfer the mechanical energy from the apparatus 3 to the skin-contacting surface in a moist or wet environment to mechanically resurface the skin.

In one embodiment, the skin-contactable element has a Durable Abrasiveness has a Durable Abrasiveness from 2 to 14, preferably from about 2.5 to about 12, more preferably from about 3 to about 10, even more preferably from about 4 to about 9.

The inventors have also surprisingly found that moderately abrasive skin-contactable elements - particularly those meeting the fixed abrasiveness criteria as specified above, have enhanced performance when used in conjunction with a mechanical tool, when the skin-contactable element also has additional properties relating to their behavior under applied compressive load. In particular, the skin-contactable element provide some displacement under an applied load, but do not displace overly so.

Displacement of generally recoverable deformation due to an applied compressive force and Compressibility are additional properties useful to characterize the skin-contactable elements. These properties may be measured according to the "Compressibility and Displacement Test" described in the "Test Methods" section, below.

As such, in one embodiment, the skin-contactable element has a Displacement from 0.15 mm to about 2.0mm, preferably from about 0.25mm to about 1mm, more preferably from about 0.25 to about 0.8mm, and most preferably from about 0.25mm to about 0.5mm.

The inventors have also surprisingly found that moderately abrasive skin-contactable elements is compressible, but not overly so. As such, in one embodiment, the skin-contactable element has a Compressibility of less than about 20%. In other embodiments, the Compressibility may be less than about 19%, or more preferably less than about 15%. Most preferably, the Compressibility may range from about 3% to about 13%.

The inventors have also noted that it is desirable for the skin-contactable element to have a thickness that is from about 0.1mm to about 20mm, preferably from about 0.5mm to about 5mm, more preferably from about 1mm to about 5mm, and most preferably from about 1.5mm to about 4.5mm. Thickness may be determined as the "Initial Thickness" in the Compressibility and Displacement Test, below. Other suitable properties of the skin-contactable element are described in commonly assigned, published U.S published patent application US20070010828 "Material for Mechanical Skin Resurfacing Techniques, hereby incorporated by reference"

The skin-contactable element 9 may further include a coating formed about or across the fibers, and, in one embodiment, formed atop the fibers 45 and atop the abrasive system as well. The coating may be least partially water-soluble such that in use, one or more ingredients within the coating dissolve in use and are transferred to the skin. 19. In one embodiment of the invention, the coating is substantially free of abrasive, such as abrasive particles that could be transferred to and embed in the skin. In one embodiment of the invention, the coating is substantially free of water (i.e., includes less than about 2%, such as less than about 0.5% of water).

The coating may be formulated for one or more of various functions. For example, the coating may provide lubrication, emolliency or and/or moisturization; mild foaming; a vehicle to deliver various benefit agents (e.g., benefit agents, drugs, and the like); or combinations thereof.

Furthermore, the coating 53 may include one or more benefit agents such as anti-acne agents, anti-wrinkle agents, anti-microbial agents, anti-fungal agents, antiinflammatory agents, topical anesthetic agents, artificial tanning agents, accelerator agents, anti-viral agents, enzyme agents, sunscreen agents, anti-oxidant agents, skin exfoliating agents, depilatory agents, and the like. Other suitable benefit agents are described in co-pending published patent application US2005-0148907, filed December 24, 2003, entitled "TREATMENT OF SKIN USING A BENEFIT AGENT AND AN APPARATUS," and co-pending patent application serial number 11/023655, filed December 28, 2004, entitled "SKIN TREATMENT ARTICLES AND METHODS, both cited previously.

### METHODS OF USE

System 1 of the present invention may be used to treat the skin, such as abrasive treatment, cleansing, or other skin treatments (e.g., acne, anti-aging, firmness, tone and texture, hair removal, body shaping/cellulite removal, and the like).

In one embodiment of the invention, the skin-contactable element is temporarily attached to the hand-held motorized apparatus. In another embodiment the skin-contactable element provides the abrasiveness and no additional abrasive is required. In another embodiment a composition (e.g., a suspension, emulsion, etc.) that includes abrasive particles is added to the skin-contactable element or to the skin to be treated.

A user grasping the apparatus and activates turns the switch on the apparatus and imparts sufficient energy to the device to cause the motor shaft to rotate and the eccentric weight coupled thereto to move in an eccentric orbit about the shaft. The vibration energy thus created from the rotating eccentric weight is transmitted to the abrasive. The vibrating abrasive contacts the skin providing benefits thereto..

The vibrating abrasive is moved across the face or other expanse of skin to be treated. For example, skin-contacting surface 11 (e.g., a substantially planar skin-contacting surface) is placed into contact with the skin to be treated. The skin-contactable element provides, for example, increased cell proliferation by abrasively treating the skin. The skin-contactable element may have incorporated therewith a formulation to provide emolliency, foam, or delivery of benefit agents to the skin. When the user is finished, the skin-contactable element may be removed and later replaced with a fresh one to provide a hygienic surface. The user may optionally rinse the treated skin after completion.

The inventors have discovered that by employing the methods of the present invention, various benefits such as cell proliferation, microdermabrasion efficacy, cleansing, and the like may be enjoyed with the added convenience of using a device that, in certain embodiments, can be made small, discrete, light, and cost-effective.

### TEST METHODS

### ABRASIVENESS TEST

"Durable Abrasiveness" is determined using the test method described below. The "Plain Abrasiveness" of a material is determined similarly to the Durable Abrasiveness, but the initial washing step is eliminated.

Five (5) samples of each of the skin-contactable elements to be tested are cut to a circular shape having a diameter of about 41 mm. Samples are individually rinsed with water in order to remove any materials such as foaming agents, oils, and emulsifiers that are readily separated from the article via contact with water. The cut samples are immersed in a bath of containing a mass of deionized water (temperature of about 35 °C) that has sufficient mass of water to be at least about 20 times the mass of the article. The article is allowed to remain in the bath for two minutes and is removed, allowed to drip for 10 seconds and then placed in another similar (fresh) water bath for two minutes, and again allowed to drip for 5 minutes. The sample is removed and allowed to dry at ambient temperature (at about 50-60% relative humidity) for a period from about 16 hours to about 72 hours. Again, this washing step is eliminated when measuring the Plain Abrasiveness.

After the sample is rinsed and dried as above, it is tested for abrasion using an abrasion testing device according to a modified version of ASTM test method D 3886-99. A suitable device is the CSI Universal Wear Tester, Model CS-226-605, available from Custom Scientific Instruments of Whippany, NJ. A sample of co-extruded spunbond/pigmented polyethylene film laminate (Clopay M18-1057, a 26 gsm laminate having (1) a 15 gsm (nominal) spunbonded polypropylene nonwoven web layer that is coextruded with (2) a 20 gsm (nominal) polyethylene film having a thickness of about 0.7 mils (0.007 inches), in which the polyethylene film surface of the laminate is corona treated, and the laminate has a target bond strength of 150 grams per inch, commercially available from Clopay Plastic Products of Mason, OH) is placed over the stage with the film oriented up, and the laminate is secured firmly against the stage with an o-ring, as supplied with the wear tester. The sample to be tested is secured on the arm above the stage such that it aligns directly on top of the stage. The sample is secured (preferably by tough double-sided tape - e.g., PERMACEL tape available from Permacel Company of East Brunswick, NJ in a manner such that the sample does not move when the tester is in operation. A 10 1b weight is loaded on the stage and the tester motor is powered. The stage simultaneously rotates and translates at a rate of about 130 cycles per minute. The number of cycles to failure is recorded as the first cycle in which the film is torn (for a pigmented, e.g., blue, film, the white spunbond readily shows through, marking the endpoint of the test. The process is repeated for the remaining samples. The average number of cycles to failure is recorded and a value for "Durable Abrasiveness" (for the washed samples) or for "Plan Abrasiveness" (for the unwashed samples) is calculated as 2000 divided by the average cycles to failure.

A standard sample, SCOTCH-BRITE Pad ("Heavy Duty Commercial Scoring Pad," # 86) is desirably run as a standard with each data set. The SCOTCH-BRITE Pad, # 86 should yield a Durable Abrasiveness value of approximately 33 +/- 4. If the operator determines a Durable Abrasiveness that falls outside this range, this signifies slight operator error, and the operator should adjust any subsequent determinations for Durable Abrasiveness by a factor that corrects for this operator error. That factor is (V/33), where V is the value determined by the operator for SCOTCH-BRITE Pad, # 86. If SCOTCH-BRITE Pad, # 86 is not available, then, as a substitute, SCOTCH-BRITE Pad ("General Purpose Commercial Scoring Pad," #96") can be run as a standard, with the expected value of Durable Abrasiveness as 14 +/- 2 and a correction factor of (V/14) if this alternative standard does not fall within the prescribed range.

The Abrasiveness value for the five samples is averaged and reported as the Durable Abrasiveness or Plain Abrasiveness value for the particular skin-contactable element.

### COMPRESSIBILITY AND DISPLACEMENT TEST

"Displacement" is determined using the following test method: for each article to be tested, five samples are cut to a size of about 41 mm diameter. One at a time, a sample is placed on a thickness gauge such as the Ames Logic Plus (model LG3601-1-04) available from BC Ames of Waltham, MA, and the sample is centered under the 55mm foot. A 0.5 oz weight is placed on the shaft and the foot is gently lowered onto the sample. The "Initial Thickness" reading is taken after the gauge is allowed to stabilize for 10 seconds. Next, the foot is lifted, the 0.5 oz. Weight is replaced with an 8 oz weight. After the gauge is allowed to stabilize for 10 seconds, the "Thickness Under Load" is recorded. The process is repeated for 10 samples. For each sample the difference between Initial Thickness and Thickness Under Load is calculated and recorded. The result for the 10 samples is averaged and recorded as the Displacement for the particular skin-contactable element.

"Compressibility" is calculated as the Displacement of a sample divided by its Initial Thickness and expressed as a percent. The result for the 10 samples is averaged and recorded as the Compressibility for the particular skin-contactable element.

### EXAMPLES

The following examples relate to skin-contactable elements of the present invention. Other embodiments of the invention can be prepared in an analogous manner by a person of ordinary skill in the art.

### Comparative Example 1

A freestanding fibrous, non-woven material (white-off-white needlepunch nonwoven material made from a blend of polyester and Lyocel® fibers; the nominal basis weight is 9 oz/yd2 (300 gsm). This fabric is a binderless nonwoven, which consists of 50% polyester fibers and 50% Lyocell® fibers).

**Table 1: Formulation of Binder Solution that is sprayed:**

| Component | wt-% |
|---|---|
| Rohm & Haas Rhoplex TR-407 | 80.00 |
| National Starch Alcogum 296W | 0.30 |
| Water (adjust as needed) | 19.30 |
| Eckart Sparkling Silver Mica | 0.20 |
| Gordon Labs Alum Oxide Code #1000999 | 0.10 |
| Blue/Purple (Sun BCD9680) Pigment Sol'n | 0.10 (est.) |
| BASF Luprintol 06-5920* (adj. pH 6.5-7.0) | 0.30 (est. only if needed) |

| | |
|---|---|
| * or Angus Chemical AMP-95 | |

Final Binder on fabric is 1.6 osy (54 gsm) and water is driven off.

The non-woven/abrasive composite was then cut into circular pads having a diameter of 41 mm. A cleansing composition was then coated across the entire top surface of the composite non-woven/abrasive system. The cleansing composition included the following ingredients:

| Trade Name | Chemical Name | % (w/w) |
|---|---|---|
| Texapon NC70 | Sodium Laureth Sulfate | 15.0000 |
| Tegobetaine F-50 | Cocamidopropyl Betaine | 6.0000 |
| Plantaren 2000 N | Decyl Glucoside | 5.0000 |
| Monateric 949J | Disodium Lauroamphodiacetate | 7.0000 |
| Atlas G-4280 | PEG-80 Sorbitan Laurate | 20.0000 |
| Glucquat 125 | Lauryl Methyl Gluceth-10Hydroxypropyldimonium Chloride | 1.0000 |
| Phenoxetol | Phenoxyethanol | 0.9000 |
| Nipa Butyl | Butyl Paraben | 0.0750 |
| Methyl Paraben | Methyl Paraben | 0.1550 |
| Propyl Paraben | Propyl Paraben | 0.1000 |
| Fragrance | Fragrance | 0.6000 |
| Citric Acid anhydrous | Citric Acid | 0.2000 |
| Carbowax PEG 400 | Polyethylene glycol | 10.9700 |
| Emery 917 | Glycerin | 33.0000 |

The PEG-80 Sorbitan Laurate and Disodium Lauroamphodiacetate were added together in a beaker and mixed until homogenous. The butylparaben, methylparaben, and propylparaben were added thereto and slowly mixed until the parabens dissolved. The PEG-8 and glucquat were then added to the beaker and mixed. The Cocamidopropyl Betaine, Sodium Laureth Sulfate, Decyl Glucoside, and Phenoxyethanol were then added and mixed. The fragrance was then added. The citric acid was then added and the ingredients mixed until the citric acid was completely dissolved. The pH was adjusted to between 6.4 and 7.2.

The skin-contactable elements 1 were mounted on an apparatus for abrasively treating the skin using a motorized device. The motorized apparatus is commercially available from Neutrogena Corporation (Los Angeles, CA) as an applicator of a micro dermabrasion system under the name, "NEUTROGENA Advanced Solutions^{™} At Home MicroDermabrasion System." ***The speed setting used was the "high" setting. The apparatus has a motor oriented with its shaft parallel to the skin-contactable element and has an eccentric mass of 4.0 grams.

The skin-contactable elements described above with respect to Example 1 were mounted on a conventional apparatus for abrasively treating the skin. A clinical assessment was performed in which 12 subjects performed a microdermabrasion treatment once every two days day in their homes. Each subject tested the systems described in Example 1 on one side of her face and Comparative Example 1 on the other side of her face. Immediately before beginning the clinical study as well after day 1, after day 7 and after day 14, clinical assessments were made. Increase in skin proliferation was determined in a manner similar to that described in US patent 5,456,260, "Fluorescence detection of cell proliferation," assigned to General Hospital Corporation, and incorporated herein by reference).

### Example 1

Skin-contactable elements were prepared identically as described for Comparative Example 1. The apparatus used in the clinical treatments was constructed by modifying the "NEUTROGENA Advanced Solutions^{™} At Home MicroDermabrasion System." Device as follows: The apparatus housing was disassembled, removing five threaded fasteners and prying apart two halves along the parting line. The original motor and motor housing were removed. An alternative motor, JameCo #175062, upon which was provided an eccentric mass of 1.4 grams. The motor was affixed to the interior surface of the apparatus using epoxy, with the motor in the identical orientation as in the comparative example. The applicator housing was reconstructed with the original threaded fasteners. The head disk was affixed with silicone epoxy.

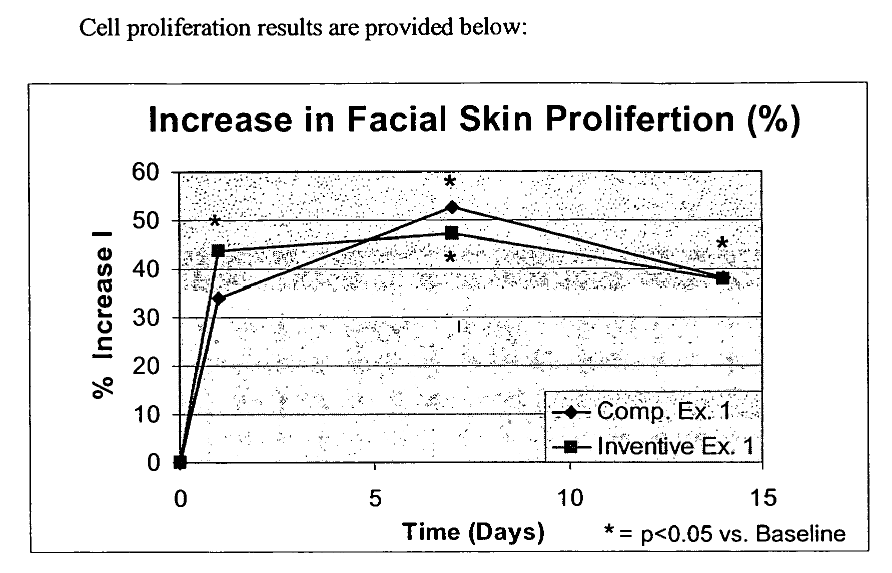

A t-test was performed to determine whether there is any statistical difference between the two treatments as well as between each of the treatments and baseline. After 1 week (3 treatments) both Comparative Example 1 and Inventive Example 1 lead to a statistical increase in cell proliferation vs. baseline. Both treatments are not statistically different at any time during the study. Surprisingly, the substitution of the micromotor (small eccentric weight) did not statistically reduce the cell proliferation provided by the treatment.

## Claims

1. A method for resurfacing the skin, comprising:
a) imparting sufficient energy to a motorized device to cause a mass of less than 4 grams to move in an eccentric orbit, thereby urging an abrasive coupled to said device to vibrate; and
b) contacting said skin with said vibrating abrasive.

2. The method of claim 1, wherein said mass is less than about 3 grams.

3. The method of claim 2, wherein said mass is less than about 2 grams.

4. The method of any preceding claim, wherein said sufficient energy urges a shaft of the motor of the apparatus to rotate, thereby urging said mass to move in said eccentric orbit.

5. The method of any preceding claim, wherein the vibrating abrasive is a vibrating skin-contactable element.

6. The method of claim 5, wherein the abrasive skin-contactable element has a Durable Abrasiveness from 2 to 14, preferably from 3 to 10.

7. The method of claim 5 or claim 6, wherein the abrasive skin-contactable element has a Displacement from about 0.2mm to about 2.0mm, preferably from about 0.25mm to about 1.0mm.

8. The method of any one of claims 5 to 7, wherein the abrasive skin-contactable element has a Compressibility of less than about 20%.

9. The method of claim 5, wherein the abrasive skin-contactable element has a Durable Abrasiveness from about 1 to about 14 and a Compressibility of from about 7% to about 18%.

10. The method of any one of claims 5 to 9, wherein the abrasive skin-contactable element comprises a porous mass.

11. The method of claim 10, wherein the abrasive skin-contactable element comprises a fibrous mass.

12. The method of claim 11, wherein the fibrous mass comprises a needlepunched web.

13. The method of claim 11 or claim 12, wherein the fibrous mass includes a bound abrasive.

14. The method of any preceding claim, further comprising:
c) moving the apparatus such that the abrasive contacts a plurality of discrete areas on the skin.
